# EUROPEAN PATENT SPECIFICATION

(11) **EP 2 644 196 B1**
(45) Date of publication and mention of the grant of the patent: **18.09.2019**
(21) Application number: 13160805.1
(22) Date of filing: 25.03.2013
(51) Int. Cl.: A61K 31/522, A61K 9/20, A61P 31/12

(54) **Pharmaceutical compositions of Entecavir**
Pharmazeutische Zusammensetzungen enthaltend Entecavir
Compositions pharmaceutiques d'entecavir

(30) Priority: 26.03.2012 TR 201203388
(43) Date of publication of application: 02.10.2013
(73) Proprietor: Arven Ilac Sanayi ve Ticaret A.S., Istinye, Istanbul 34460 (TR)
(72) Inventor: Cifter, ÜMIT, 34460 Istanbul (TR); Türkyilmaz, Ali, 34460 Istanbul (TR); Mutlu, Onur, 34460 Istanbul (TR)
(74) Representative: Sevinç, Erkan

(56) References cited:
- EP-A1- 2 508 172
- WO-A1-01/64221
- WO-A1-2011/076412
- WO-A2-2013/072937
- US-A1- 2010 221 323
- Bristol-Myers Squibb: "Baraclude Tablets", , 15 April 2011 (2011-04-15), pages 1-4, XP055045636, Retrieved from the Internet: URL:http://www.bmsa.com.au/PublishingImage s/products/pdf/Entecavir monohydrate.pdf [retrieved on 2012-11-26]
- Jrs Pharma: "Prosolv EASYtab", , 1 January 2010 (2010-01-01), pages 1-2, XP055045597, Retrieved from the Internet: URL:http://www.jrspharma.de/Pharma/wDeutsc h/produktinfo/productinfo_prosolv_easytab. shtml [retrieved on 2012-11-26]

## Description

### Technical Aspect

This invention is related to a novel pharmaceutical composition of entecavir monohydrate, comprising sodium starch glycolate and sodium stearyl fumarate with at least one pharmaceutically acceptable excipient, for oral administration, for their preparation and use thereof.

### Background of the Invention

Entecavir is a guanosine nucleoside analogue with selective activity against Hepatitis B virus (HBV). The chemical name for entecavir is 2-amino-1,9-dihydro-9-[(1S,3R,4S)-4hydroxy-3-(hydroxymethyl)-2-methylenecyclopentyl]-6H-purin-6-one, monohydrate. Entecavir has the following structural formula shown in below:

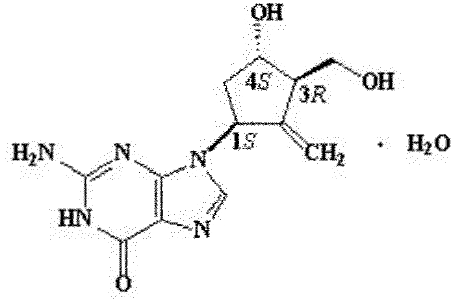

Entecavir is marketed under the name of BARACLUDE, available for oral administration as tablet and oral solutions in strengths of 0.5 mg and 1 mg of entecavir, and it is indicated for the treatment of chronic hepatitis B virus infection in adults.

Entecavir and its use in treating hepatitis B are disclosed in U.S.Patent 5,206,244. Methods for the synthesis of entecavir are disclosed in WO 98/09964.

This invention relates to pharmaceutical compositions for oral administration containing a low dose entecavir. However, it is known that low dose pharmaceutical compositions have the problem of obtaining adequate content uniformity. Because it is difficult to homogenize the low active ingredient in final dosage form and some other difficulties may occur compressing them and this may cause inadequate content uniformity of the final dosage forms. Various processes can be used to get over this problem but they are more complicated and time consuming.

It is also known in the pharmaceutical field that, when formulating a composition with low dose pharmaceutical active ingredients for administration to those in need of therapy, the challenge is to ensure an even distribution of the pharmaceutically active ingredients throughout the pharmaceutical excipients to ensure a proper dosage and homogeneity. Therefore, it would be desirable to provide solid oral dosage forms that have an adequate content uniformity and that disperse well upon oral administration. Furthermore, it would be also desirable to provide the solid dosage formulation which can be easily processed without requiring additional expensive and difficult manufacturing technologies.

Low dose entecavir formulations and methods to prepare them are also disclosed in WO01/64221 and in WO2011/076412.

Thus, there is need in the art for pharmaceutical compositions of entecavir for oral administration, which has an adequate content uniformity causing a good dispersion upon oral administration wich is easily processes and has high bioavailability and improved stability for their preparation and use thereof.

### Summary of the Invention

The present invention provides a novel pharmaceutical composition of entecavir monohydrate, comprising sodium starch glycolate and sodium stearyl fumarate with at least one pharmaceutically acceptable excipient which overcomes the above described problems in prior art and has additive advantages over them, with the provisio that said composition doesn't comprise povidone or crospovidone.

The main object of the present invention is to obtain adequate content uniformity of low dosage forms of entecavir monohydrate with using adequate excipients and improved processes.

There is also provided an improved process for preparing the entecavir which has a low dose active ingredient. It is difficult to prepare entecavir formulations with a simple process such as direct compression because of having low doses of entecavir such as from 0.01 to 2.0 mg.

Another object of this invention is to provide an improved stability with high bioavailability of pharmaceutical compositions of entecavir monohydrate.

A further embodiment of the invention is the pharmaceutical composition of entecavir monohydrate, wherein the weight ratio of sodium starch glycolate to sodium stearyl fumarate is 1:5 to 50:1 by weight, preferably it is 1:2 to 25:1 by weight, more preferably 1:1 to 10:1 by weight.

As a further embodiment, the pharmaceutical composition further comprising hydroxypropyl methylcellulose.

In another embodiment, the weight ratio of hydroxypropyl methylcellulose to entecavir monohydrate is 50:1 to 1:10 by weight, preferably it is 25:1 to 1:5 by weight, more preferably 15:1 to 1:1 by weight.

As a further embodiment of the invention, at least one pharmaceutically acceptable excipient is a filler which is selected from the group comprising microcrystalline cellulose, mannitol, starch, lactose, sugars, sorbitol, sucrose and mixtures thereof. Preferably the fillers are microcrystalline cellulose, mannitol and starch.

As a further embodiment of the invention, at least one pharmaceutically acceptable excipient is a glidant which is selected from the group comprising colloidal silicon dioxide, talc, aluminium silicate and mixtures thereof; preferably the glidant is colloidal silicon dioxide.

Yet another embodiment of the invention, entecavir monohydrate is present in an amount of 0.01 to 5.0% by weight of total composition; preferably it is 0.1 to 2.0% by weight of total composition.

As a further embodiment, the invention provides the pharmaceutical composition of entecavir monohydrate comprising;
a. 0.01 - 5.0 % of entecavir monohydrate
b. 1.0 - 30 % of starch
c. 1.0 - 30 % of sodium starch glycolate
d. 10.0 - 80 % of microcrystalline cellulose
e. 5.0 - 30 % of mannitol
f. 0.10 - 10.0 % of hydroxypropyl methyl cellulose
g. 0.10 - 5.0 % of sodium stearyl fumarate
h. 0.01 - 2.0 % of colloidal silicon dioxide

As a further embodiment, the pharmaceutical composition is in the form of tablets, capsules, powders, sachets; preferably the final dosage form is in the form of a tablet.

As a further embodiment, the invention provides the final dosage form which has a content uniformity of less than 2.0% RSD, preferably less than 1.0 % RSD.

As a further embodiment, the invention provides a direct compression process for preparing the pharmaceutical composition of entecavir monohydrate comprising the following steps;
a. blending entecavir monohydrate, microcrystalline cellulose, mannitol, sodium starch glycoloate and hydroxypropylmethylcellulose progressively, wherein the blending time is preferably 20 min.,
b. adding sodium stearyl fumarate to the powder mixture above and blending progressively for about 5 min.
c. compressing the final powder mixture to form tablets,
d. coating the tablets

As a further embodiment, the invention provides a granulation process for preparing the pharmaceutical composition of entecavir monohydrate comprising the following steps;
a. blending entecavir monohydrate, 1/3 of microcrystalline cellulose and hydroxypropylmethylcelluloses progressively,
b. compacting the blended mixture,
c. adding mannitol, 80% of colloidal silicon dioxide, 2/3 of microcrystalline cellulose and sodium starch glycoloate to this mixture of step b., and further progressive blending for 15 min.,
d. adding sodium stearyl fumarate and rest of the colloidal silicon dioxide and blending this mixture until obtaining a homogenous powder mixture,
e. compressing the blended mixture to form tablets
f. coating the tablets

As a further embodiment, the invention provides a wet granulation process for preparing the pharmaceutical composition of entecavir monohydrate comprising the following steps;
a. adding hydroxypropyl methylcellulose, starch and 20% by weight of mannitol and entecavir in a granulator and blending them for 10 min.
b. adding water to this mixture and blending to form granules
c. sieving and drying the wet granules,
d. adding microcrystalline cellulose, sodium starch glycolate and rest of the mannitol and blending them for 10 min.
e. adding colloidal silicon dioxide and sodium stearyl fumarate to this mixture and blending them until obtaining a homogenous powder mixture,
f. compressing the blended mixture to form tablets
g. coating the tabets

Yet, another embodiment of the invention is the pharmaceutical composition of entecavir monohydrate for use in the treatment of hepatitis B virus infection.

Further advantages and embodiments of the present invention will become apparent from the following description

### Detailed Description of the Invention

It is known that entecaivr has a very low dose in pharmaceutical compositions and have the problem of obtaining adequate content uniformity. Because it is difficult to homogenize in final dosage forms and some other difficulties may occur when compressing them and this may cause inadequate content uniformity and poor dissolution profile of the final dosage forms. To avoid these problems generaly polyvinylpyrrolidone (povidone) and/or cross-lynked polyvinylpyrrolidone (crospovidone) is used to obtain a good dispersion, but this is not enough alone and particle size reduction of the active ingredient is also preffered.

However, using povidone and/or crospovidone in entecavir formulations may cause stability problems because of their hygroscopic properties, they are more hygroscopic other than the other disintegrants especially when used in high amounts to obtain a good dispersion for the cotent uniformity of the final dosage form. Also because of binding properties of povidone the dissolution may be effected in negative way.

Accordingly, particle size reduction (less than about 50 µm or even more such as less than about 10 µm) may not always effective enough for increasing the dissolution rate of entecavir. Many low-dose drugs such as entecavir have a strong tendency to agglomerate during the manufacturing process into larger particles with an overall decrease in effective surface area. The technical problem of particle size reduction may occur during the manufacturing and tabletting procedure such as agglomeration and bad flowabilty.

We have found that, solid oral dosage forms such as tablets, when prepared with sodium starch glycolate have good storage properties. Sodium starch glycolate has advantages over other unmodified starches because unmodified starch has poor flow characteristics and tends to increase tablet friability and capping if used in high concentrations. Additionally, the improved flow and lubricity characteristics of sodium starch glycolate can impart further benefit to the formulation in a very cost-effective manner.

It is also observed that, sodium stearyl fumarate is less sensitive to overblending and compressibility than other lubricants, this gives the advantage to the present formulations to have better compressibility and robustness to overblending without causing poor dissolution profiles.

Surprisingly it is found that when sodium starch glycolate and sodium stearyl fumarate is used with entecavir monohydrate, the pharmaceutical compositions of this invention has better storage stability and better compressibility with less friability. Thus, an adequate content uniformity is achieved in the final dosage forms, wherein the content uniformity is less than 2.0 % RSD (Relative Standard Deviation), preferably it is less than 1.0 % RSD. Herein, content uniformity test is determined by calculating RSD (Relative Standard Deviation) value of 10 tablets (According to European Pharmacopea 7.0, page.315-317, 2.9.40. Uniformity Of Dosage Units).

As a further embodiment, the friability is less than 1.0 %.

Another object of this present invention is a pharmaceutical composition of entecavir monohydrate further comprising hydroxyl propyl methyl cellulose (HPMC). Accordingly, a synergistic effect is observed over the distribution of the low doses of entecavir monohydrate throughout other pharmaceutical excipients when HPMC is used in a specific weight ratio with entecavir which is 1:5 to 50:1 (w/w), preferably the ratio is 1:2 to 25:1 (w/w), more preferably the ratio is 1:1 to 10:1 (w/w).

In a further embodiment, the pharmaceutical composition of this invention comprises entecavir having a median particle size, (d₅₀) greater than 50 µm, preferably greater than 80 µm, more preferably greater than 100 µm and having particle size of d₉₀ greater than 100 µm, preferably greater than 150 µm.

As used here in, "particle size distribution" means the cumulative volume size distrubition as tested by any conventionally accepted method such as the laser diffraction method. "median particle size" means, d₅₀, the size at which 50% by volume of the particles are finer and "d₉₀" means that the size at which %90 by volume of the particles are finer.

As a further embodiment of the invention, it is even possible to prepare tablets by direct compression without the requirement of previous granulation of the active ingredient, entecavir. Likewise the granulation is possible as well, because it is found that when the formulation is prepared by the excipients described above improve the formulation to an easily processed formulation. Accordingly a progressive blending technique is used to improve the distribution of the low dose active ingredient, entecavir.

Progressive blending is a technique used in mixing two excipients of unequal quantities, where one begins with the smallest quantity and adds an equal quantity of the excipient having the larger amount; process continues until all of the ingredients are used.

Accordingly, it is even possible to prepare tablets by direct compression without the requirement of previous granulation of the active ingredient into larger particles in order to increase the flow properties. Likewise the granulation is possible as well, because the flow behaviour of the entecavir of a particle size greater than 50 micron or more such as 100 micron, and a narrow particle size distribution facilitates the granulation process when combined with progressive blending. Thus, it is possible to prepare pharmaceutical compositions with the entecavir of the present invention in a fast and cost efficient manner.

The preferred direct compression process of the present invention for preparing the pharmaceutical composition of entecavir monohydrate comprises the following steps;
a. blending entecavir monohydrate, microcrystalline cellulose, mannitol, sodium starch glycoloate and hydroxypropylmethylcellulose progressively, wherein the blending time is preferably 20 min.,
b. adding sodium stearyl fumarate to the powder mixture above and blending progressively for about 5 min.
c. compressing the final powder mixture to form tablets,
d. coating the tablets

The preferred granulation process of the present invention for preparing the pharmaceutical composition of entecavir monohydrate comprises the following steps;
a. blending entecavir monohydrate, 1/3 of microcrystalline cellulose and hydroxypropylmethylcelluloses progressively,
b. compacting the blended mixture,
c. adding mannitol, 80% of colloidal silicon dioxide, 2/3 of microcrystalline cellulose and sodium starch glycoloate to this mixture of step b., and further progressive blending for 15 min.,
d. adding sodium stearyl fumarate and rest of the colloidal silicon dioxide and blending this mixture until obtaining a homogenous powder mixture,
e. compressing the blended mixture to form tablets
f. coating the tablets

The preffered wet granulation process for preparing the pharmaceutical composition of entecavir monohydrate comprising the following steps;
a. adding hydroxypropyl methylcellulose, starch and 20% by weight of mannitol and entecavir in a granulator and blending them for 10 min.
b. adding water or water/alcohol mixture to this mixture and blending to form granules,
c. sieving and drying the wet granules,
d. adding microcrystalline cellulose, sodium starch glycolate and rest of the mannitol and blending them for 10 min.
e. adding colloidal silicon dioxide and sodium stearyl fumarate to this mixture and blending them until obtaining a homogenous powder mixture,
f. compressing the blended mixture to form tablets
g. coating the tabets

One of the preffered embodiments of this invention is using high shear granulator during wet granulation process instead of fluid bed dryer to get over some technical problems such as described below;
a. to obtain a good flowability and compressibility of the final blended powder mixture,
b. to obtain hard granules at the end of the process which lessens the dustiness, and this provides less friable granules,
c. narrowing the particule size distribution of the powder mixtures,
d. to ensure an even distribution of the pharmaceutically active ingredient throughout the pharmaceutical excipients to ensure a proper dosage and homogeneity and this has an additive effect over content uniformity,
e. using less binder solution and this makes the timing of the process shorter,
f. to improve dissolution characteristics of the final dosage forms.

As a further embodiment, the pharmaceutical compositions according to the present invention may further comprise one or more other active ingredients such as tenofovir.

In a further embodiment, the pharmaceutical compositions of this invention are suitable for the treatment of hepatit - B and complications or disorders associated therewith.

As a further embodiment, the pharmaceutical compositions of entecavir monohydrate comprise at least one pharmaceutically acceptable excipient. Suitable pharmaceutically acceptable excipients comprise but are not limited to disintegrants, fillers, binders, lubricants and glidants.

Suitable disintegrants other than sodium starch glycolate, may comprise but not limited to croscarmellose sodium, alginic acid, sodium alginate, microcrystalline cellulose, carboxymethylcellulose sodium, docusate sodium, low-substituted hydroxypropyl cellulose, magnesium aluminum silicate, poloxamer, sodium glycine carbonate and mixtures thereof.

Suitable fillers may comprise but not limited to starch, microcrystalline cellulose, mannitol, lactose, dibasic calcium phosphate dihydrate, polysaccharides, sugars, sorbitol, maltitol, sucrose, maltodextrin, sucrose-maltodextrin coprecipitate, Xylitol and mixtures thereof. The most preffered fillers are starch, microcrystalline cellulose and mannitol.

Suitable binders other than hydroxyl propyl methyl cellulose, may comprise but not limited to hydroxypropyl cellulose, carboxy methyl cellulose, methyl cellulose, gelatin, natural gums, polymethycrylate and mixtures thereof.

Suitable lubricants other than sodium stearyl fumarate may comprise but not limited to polyethylene glycol, stearic acid, metal stearates, talc, boric acid, sodium chloride benzoate and acetate, sodium or magnesium lauryl sulfate and mixtures thereof.

Suitable glidants other than colloidal silicon dioxide, may comprise but not limited to talc, aluminium silicate, calcium silicate, magnesium silicate, magnesium carbonate, magnesium oxide and mixtures thereof. The most preffered glidant is colloidal silicon dioxide.

This invention is further defined by reference to the following examples. Although the examples are not intended to limit the scope of the present invention, they should be considered in the light of the description detailed above.

### Example 1 : Entecavir monohydrate tablet formulation

| **Ingredients** | **Amount mg** |
|---|---|
| entecavir monohydrate | 1.00 |
| starch | 60.0 |
| sodium starch glycolate | 60.0 |
| Microcrystalline cellulose (pH-102) | 180.0 |
| Mannitol | 70.0 |
| Hydroxypropyl methylcellulose | 10.0 |
| Sodium stearyl fumarate | 12.0 |
| Colloidal silicon dioxide | 2.0 |
| **Total tablet weight** | **395.0** |

The formulations of these examples can be manufactured according to the processes described detailed above in the description and tablet formulations further comprise a film coating in an amount of 2.5 % which is disclosed in detail below.

### Coating with:

| | |
|---|---|
| HPMC - Methocel | 62,50 % |
| Titanium dioxide | 28.75 % |
| Poylethyleneglycol 400 | 6.25 % |
| FD&C yellow & sunset yellow | 2.50 % |

### Example 2:

The pharmaceutical composition of this present invention (Ex.1) was tested for its dissolution profile against an entecavir composition (see the Referance Product as comperative formulation below) including povidone and crospovidone instead of sodium starch glycolate and sodium stearyl fumarate in 1000 ml of 0.05 M phosphate buffer at pH 6.8, at 37ºC using a USP paddle II method rotating at 50 RPM. According to the results the entecavir formulation of the present invention (Ex.1) has a high dissolution profile at least 85% in 15 min., while the comperative formulation has a poor dissolution profile lower than 85% in 15 min. Thus, the above mentioned excipients, especially sodium starch glycolate and sodium stearyl fumarate increase the solubility of entecavir (See Table 1).

**Table 1 - Dissolution Profiles**

| | Ex. 1 | Reference Product as comperative formulation |
|---|---|---|
| 0 | 0 | 0 |
| 5 | 78 | 65 |
| 10 | 91 | 71 |
| 15 | 95 | 80 |
| 20 | 99 | 89 |

### Example 3:

The pharmaceutical composition of this present invention (Ex.1) was tested for its content uniformity against the same comperative entecavir formulation used in Ex.2 (see the Referance Product as comperative formulation below). According to the results the present formulation of Ex.1 has a content uniformity which is less than 0.50% RSD while the comperative formulation has a content uniformity higher than 0.50% RSD even 1.0 % RSD (See Table 2). Herein, content uniformity test is determined by calculating RSD (Relative Standard Deviation) value of 10 tablets.

### Referance Product as comperative formulation used in Ex 2 and 3 :

Entecavir monohydrate as active ingredient and lactose monohydrate, crospovidone, povidone, microcrystalline cellulose, colloidal silicon dioxide, magnesium stearate as inactive ingredients.

**Table 2 - Content Uniformity Results**

| | Ex. 1 | Reference Product as comperative formulation |
|---|---|---|
| 1 | 100,14 | 105,19 |
| 2 | 99,8 | 104,18 |
| 3 | 99,27 | 103,59 |
| 4 | 99,37 | 103,66 |
| 5 | 99,78 | 100,67 |
| 6 | 99,25 | 101,73 |
| 7 | 100,39 | 104,30 |
| 8 | 100,5 | 105,91 |
| 9 | 99,68 | 102,18 |
| 10 | 99,18 | 101,04 |
| Mean | 99,74 | 103,2 |
| SD | 0,48 | 1,7 |
| RSD | 0,48 | 1.6 |

## Claims

1. A pharmaceutical composition of entecavir monohydrate, comprising sodium starch glycolate and sodium stearyl fumarate with at least one pharmaceutically acceptable excipient, with the provisio that said composition doesn't comprise povidone or crospovidone.

2. The pharmaceutical composition of entecavir monohydrate according to the claim 1, wherein the weight ratio of sodium starch glycolate to sodium stearyl fumarate is 1:5 to 50:1 by weight, preferably it is 1:2 to 25:1 by weight, more preferably 1:1 to 10:1 by weight.

3. The pharmaceutical composition of entecavir monohydrate according to claim 1, further comprising hydroxypropyl methylcellulose.

4. The pharmaceutical composition of entecavir monohydrate according to the claim 1 and 3, wherein the weight ratio of hydroxypropyl methylcellulose to entecavir monohydrate is 50:1 to 1:10 by weight, preferably it is 25:1 to 1:5 by weight, more preferably 15:1 to 1:1 by weight.

5. The pharmaceutical composition of entecavir monohydrate according to the claim 1, wherein at least one pharmaceutically acceptable excipient is a filler which is selected from the group comprising microcrystalline cellulose, mannitol, starch, lactose, sugars, sorbitol, sucrose and mixtures thereof.

6. The pharmaceutical composition of entecavir monohydrate according to the claim 5, wherein the fillers are microcrystalline cellulose, mannitol and starch.

7. The pharmaceutical composition of entecavir monohydrate according to the claim 1, wherein at least one pharmaceutically acceptable excipient is a glidant which is selected from the group comprising colloidal silicon dioxide, talc, aluminium silicate and mixtures thereof; preferably the glidant is colloidal silicon dioxide.

8. The pharmaceutical composition of entecavir monohydrate according to claim 1, wherein entecavir monohydrate is present in an amount of 0.01 to 5.0% by weight of total composition; preferably it is 0.1 to 2.0% by weight of total composition.

9. The pharmaceutical composition of entecavir monohydrate according to claims 1 to 8, comprising;
a. 0.01 - 5.0 % of entecavir monohydrate,
b. 1.0 - 30 % of starch,
c. 1.0 - 30 % of sodium starch glycolate,
d. 10.0 - 80 % of microcrystalline cellulose,
e. 5.0 - 30 % of mannitol,
f. 0.10 - 10.0 % of hydroxypropyl methyl cellulose,
g. 0.10 - 5.0 % of sodium stearyl fumarate,
h. 0.01 - 2.0 % of colloidal silicon dioxide.

10. The pharmaceutical composition of entecavir monohydrate according to any preceding claims, wherein the pharmaceutical composition is in the form of tablets, capsules, powders, sachets; preferably the final dosage form is in the form of a tablet.

11. The pharmaceutical composition of entecavir monohydrate according to any preceding claim, wherein the final dosage form has a content uniformity of less than 2.0% RSD , preferably less than 1.0 % RSD as determined according to European Pharmacopea 7.0, Uniformity of Dosage Units.

12. A direct compression process for preparing the pharmaceutical composition of entecavir monohydrate according to any preceding claims, comprising the following steps;
a. blending entecavir monohydrate, microcrystalline cellulose, mannitol, sodium starch glycoloate and hydroxypropylmethylcellulose progressively, wherein the blending time is preferably 20 min.,
b. adding sodium stearyl fumarate to the powder mixture above and blending progressively for about 5 min.
c. compressing the final powder mixture to form tablets,
d. coating the tablets

13. A granulation process for preparing the pharmaceutical composition of entecavir monohydrate according to any preceding claims, comprising the following steps;
a. blending entecavir monohydrate, 1/3 of microcrystalline cellulose and hydroxypropylmethylcelluloses progressively,
b. compacting the blended mixture,
c. adding mannitol, 80% of colloidal silicon dioxide, 2/3 of microcrystalline cellulose and sodium starch glycoloate to this mixture of step b., and further progressive blending for 15 min.,
d. adding sodium stearyl fumarate and rest of the colloidal silicon dioxide and blending this mixture until obtaining a homogenous powder mixture,
e. compressing the blended mixture to form tablets
f. coating the tablets

14. A wet granulation process for preparing the pharmaceutical composition of entecavir monohydrate according to any preceding claims, comprising the following steps;
a. adding hydroxypropyl methylcellulose, starch and 20% by weight of mannitol and entecavir in a granulator and blending them for 10 min.
b. adding water to this mixture and blending to form granules
c. sieving and drying the wet granules,
d. adding microcrystalline cellulose, sodium starch glycolate and rest of the mannitol and blending them for 10 min.
e. adding colloidal silicon dioxide and sodium stearyl fumarate to this mixture and blending them until obtaining a homogenous powder mixture,
f. compressing the blended mixture to form tablets
g. coating the tablets

15. The pharmaceutical composition of entecavir monohydrate according to any preceding claims for use in the treatment of hepatitis B virus infection.

## Patentansprüche

1. Pharmazeutische Zusammensetzung von Entecavir-Monohydrat, die Natriumstärkeglykolat und Natriumstearylfumarat mit zumindest einem pharmazeutisch akzeptablen Exzipienten aufweist, mit der Maßgabe, dass die Zusammensetzung kein Povidon oder Crospovidon aufweist.

2. Pharmazeutische Zusammensetzung von Entecavir-Monohydrat gemäß Anspruch 1, wobei das Gewichtsverhältnis von Natriumstärkeglykolat zu Natriumstearylfumarat 1:5 bis 50:1 Gewichtseinheiten, vorzugsweise 1:2 bis 25:1 Gewichtseinheiten, weiter bevorzugt 1:1 bis 10:1 Gewichtseinheiten beträgt.

3. Pharmazeutische Zusammensetzung von Entecavir-Monohydrat gemäß Anspruch 1, die ferner Hydroxypropyl-Methylzellulose aufweist.

4. Pharmazeutische Zusammensetzung von Entecavir-Monohydrat gemäß Anspruch 1 und 3, wobei das Gewichtsverhältnis von Hydroxypropyl-Methylzellulose zu Entecavir-Monohydrat 50:1 bis 1:10 Gewichtseinheiten, vorzugsweise 25:1 bis 1:5 Gewichtseinheiten, weiter bevorzugt 15:1 bis 1:1 Gewichtseinheiten beträgt.

5. Pharmazeutische Zusammensetzung von Entecavir-Monohydrat gemäß Anspruch 1, wobei zumindest ein pharmazeutisch akzeptabler Exzipient ein Füllstoff ist, der ausgewählt ist aus der Gruppe aufweisend mikrokristalline Zellulose, Mannitol, Stärke, Laktose, Zucker, Sorbitol, Saccharose und Mischungen hieraus.

6. Pharmazeutische Zusammensetzung von Entecavir-Monohydrat gemäß Anspruch 5, wobei die Füllstoffe mikrokristalline Zellulose, Mannitol und Stärke sind.

7. Pharmazeutische Zusammensetzung von Entecavir-Monohydrat gemäß Anspruch 1, wobei zumindest ein pharmazeutisch akzeptabler Exzipient ein Gleitmittel ist, das ausgewählt ist aus der Gruppe aufweisend kolloidales Siliziumdioxid, Talkum, Aluminiumsilikat und Mischungen hieraus; vorzugsweise ist das Gleitmittel kolloidales Siliziumdioxid.

8. Pharmazeutische Zusammensetzung von Entecavir-Monohydrat gemäß Anspruch 1, wobei Entecavir-Monohydrat in einer Menge von 0,01 bis 5,0 Gew.-% der gesamten Zusammensetzung vorliegt; vorzugsweise liegt es bei 0,1 bis 2,0 Gew.-% der gesamten Zusammensetzung.

9. Pharmazeutische Zusammensetzung von Entecavir-Monohydrat gemäß den Ansprüchen 1 bis 8, die Folgendes aufweist;
a. 0,01 - 5,0 % Entecavir-Monohydrat,
b. 1,0 - 30 % Stärke,
c. 1,0 - 30 % Natriumstärkeglykolat,
d. 10,0 - 80 % mikrokristalline Zellulose,
e. 5.0 - 30 % Mannitol,
f. 0,10 - 10,0 % Hydroxypropyl-Methylzellulose,
g. 0,10 - 5,0 % Natriumstearylfumarat,
h. 0,01 - 2,0 % kolloidales Siliziumdioxid.

10. Pharmazeutische Zusammensetzung von Entecavir-Monohydrat gemäß einem der vorherigen Ansprüche, wobei die pharmazeutische Zusammensetzung in Form von Tabletten, Kapseln, Puder, Beuteln vorliegt; vorzugsweise ist die Form der finalen Dosierung eine Tablettenform.

11. Pharmazeutische Zusammensetzung von Entecavir-Monohydrat gemäß einem der vorherigen Ansprüche, wobei die Form der finalen Dosierung eine Gehaltseinheitlichkeit von weniger als 2,0% RSD, vorzugsweise von weniger als 1,0 % RSD aufweist, gemessen gemäß European Pharmacopea 7,0, Uniformity of Dosage Units.

12. Direktkompressionsverfahren zur Herstellung der pharmazeutischen Zusammensetzung von Entecavir-Monohydrat gemäß einem der vorherigen Ansprüche, das folgende Schritte aufweist;
a. schrittweises Vermengen von Entecavir-Monohydrat, mikrokristalliner Zellulose, Mannitol, Natriumstärkeglykoloat und Hydroxypropyl-Methylzellulose, wobei die Zeit der Vermengung vorzugsweise 20 min. beträgt,
b. Zugabe von Natriumstearylfumarat zu der obigen Pulvermischung und schrittweises Vermengen für etwa 5 min.,
c. Komprimieren der finalen Pulvermischung, um Tabletten zu formen,
d. Beschichten der Tabletten.

13. Granulierungsverfahren zur Herstellung der pharmazeutischen Zusammensetzung von Entecavir-Monohydrat gemäß einem der vorherigen Ansprüche, das die folgenden Schritte aufweist;
a. schrittweises Vermengen von Entecavir-Monohydrat, 1/3 von mikrokristalliner Zellulose und Hydroxypropyl-Methyzellulose,
b. Kompaktieren der vermengten Mischung,
c. Zugabe von Mannitol, 80% kolloidalem Siliziumdioxid, 2/3 mikrokristalliner Zellulose und Natriumstärkeglykolat zu dieser Mischung aus Schritt b., und weiter schrittweises Vermengen für 15 min.,
d. Zugabe von Natriumstearylfumarat und dem Rest des kolloidalen Siliziumdioxids und Vermengen dieser Mischung bis zum Erhalt einer homogenen Pulvermischung,
e. Komprimieren der vermengten Mischung, um Tabletten zu formen,
f. Beschichten der Tabletten.

14. Feuchtgranulierungsverfahren zur Herstellung der pharmazeutischen Zusammensetzung von Entecavir-Monohydrat gemäß einem der vorherigen Ansprüche, das die folgenden Schritte aufweist;
a. Zugabe von Hydroxyproply-Methylzellulose, Stärke und 20 Gew.-% Mannitol und Entecavir in einen Granulator und Vermengen derselben für 10 min.
b. Zugabe von Wasser zu dieser Mischung und Vermengen, um Granulat zu formen,
c. Sieben und Trocknen des feuchten Granulats,
d. Zugabe von mikrokristalliner Zellulose, Natriumstärkeglykolat und dem Rest des Mannitols und Vermengen derselben für 10 min.
e. Zugabe von kolloidalen Siliziumdioxid und Natriumstearylfumarat zu dieser Mischung und Vermengen derselben, bis zum Erhalt einer homogenen Pulvermischung,
f. Komprimieren der vermengten Mischung, um Tabletten zu formen,
g. Beschichten der Tabletten.

15. Pharmazeutische Zusammensetzung von Entecavir-Monohydrat gemäß einem der vorherigen Ansprüche zur Verwendung in der Behandlung einer Infektion mit dem Hepatitis-B-Virus.

## Revendications

1. Composition pharmaceutique de monohydrate d'entécavir, comprenant du glycolate d'amidon sodique et du fumarate de sodium et de stéaryle avec au moins un excipient pharmaceutiquement acceptable, à la condition que ladite composition ne comprenne pas de povidone ou de crospovidone.

2. Composition pharmaceutique de monohydrate d'entécavir selon la revendication 1, dans laquelle le rapport en poids du glycolate d'amidon sodique au fumarate de sodium et de stéaryle est de 1:5 à 50:1 en poids, de préférence est de 1:2 à 25:1 en poids, de façon davantage préférée de 1:1 à 10:1 en poids.

3. Composition pharmaceutique de monohydrate d'entécavir selon la revendication 1, comprenant en outre de l'hydroxypropyl méthylcellulose.

4. Composition pharmaceutique de monohydrate d'entécavir selon l'une des revendications 1 et 3, dans laquelle le rapport en poids de l'hydroxypropyl méthylcellulose au monohydrate d'entécavir est de 50:1 à 1:10 en poids, de préférence est de 25:1 à 1:5 en poids, de façon davantage préférée de 15:1 à 1:1 en poids.

5. Composition pharmaceutique de monohydrate d'entécavir selon la revendication 1, dans laquelle au moins un excipient pharmaceutiquement acceptable est une charge qui est choisie dans le groupe comprenant la cellulose microcristalline, le mannitol, l'amidon, le lactose, les sucres, le sorbitol, le sucrose et leurs mélanges.

6. Composition pharmaceutique de monohydrate d'entécavir selon la revendication 5, dans laquelle les charges sont la cellulose microcristalline, le mannitol et l'amidon.

7. Composition pharmaceutique de monohydrate d'entécavir selon la revendication 1, dans laquelle au moins un excipient pharmaceutiquement acceptable est un agent de glissement qui est choisi dans le groupe comprenant le dioxyde de silicium colloïdal, le talc, le silicate d'aluminium et leurs mélanges ; de préférence l'agent de glissement est le dioxyde de silicium colloïdal.

8. Composition pharmaceutique de monohydrate d'entécavir selon la revendication 1, dans laquelle le monohydrate d'entécavir est présent dans une quantité de 0,01 à 5,0 % en poids de la composition totale ; de préférence est de 0,1 à 2,0 % en poids de la composition totale.

9. Composition pharmaceutique de monohydrate d'entécavir selon l'une des revendications 1 à 8, comprenant :
a. 0,01-5,0 % de monohydrate d'entécavir ;
b. 1,0-30 % d'amidon ;
c. 1,0-30 % de glycolate d'amidon sodique ;
d. 10,0-80 % de cellulose microcristalline ;
e. 5,0-30 % de mannitol ;
f. 0,10-10,0 % d'hydroxypropyl méthyl cellulose ;
g. 0,10-5,0 % de fumarate de sodium et de stéaryle ;
h. 0,01-2,0 % de dioxyde de silicium colloïdal.

10. Composition pharmaceutique de monohydrate d'entécavir selon l'une quelconque des revendications précédentes, dans laquelle la composition pharmaceutique est sous la forme de comprimés, de capsules, de poudres, de sachets ; de préférence, la forme posologique finale est sous la forme d'un comprimé.

11. Composition pharmaceutique de monohydrate d'entécavir selon l'une quelconque des revendications précédentes, dans laquelle la forme posologique finale a une uniformité de contenu de moins de 2,0 % d'écart-type relatif (ETR), de préférence de moins de 1,0 % d'ETR tel que déterminé conformément à la Pharmacopée Européenne 7.0, Uniformité de Formes Posologiques.

12. Procédé de compression directe pour préparer la composition pharmaceutique de monohydrate d'entécavir selon l'une quelconque des revendications précédentes, comprenant les étapes suivantes :
a. mélanger progressivement du monohydrate d'entécavir, de la cellulose microcristalline, du mannitol, du glycolate d'amidon sodique et de l'hydroxypropylméthylcellulose, le temps de mélange étant, de préférence, de 20 min ;
b. ajouter du fumarate de sodium et de stéaryle au mélange de poudres ci-dessus et mélanger progressivement pendant environ 5 min ;
c. comprimer le mélange de poudres final pour former des comprimés ;
d. enrober les comprimés.

13. Procédé de granulation pour préparer la composition pharmaceutique de monohydrate d'entécavir selon l'une quelconque des revendications précédentes, comprenant les étapes suivantes :
a. mélanger progressivement du monohydrate d'entécavir, 1/3 de cellulose microcristalline et de l'hydroxypropylméthylcellulose ;
b. compacter le mélange mélangé ;
c. ajouter du mannitol, 80 % de dioxyde de silicium colloïdal, 2/3 de cellulose microcristalline et du glycolate d'amidon sodique à ce mélange de l'étape b., et encore mélanger progressivement pendant 15 min ;
d. ajouter du fumarate de sodium et de stéaryle et le reste du dioxyde de silicium colloïdal et mélanger ce mélange jusqu'à obtention d'un mélange de poudres homogène ;
e. comprimer le mélange mélangé pour former des comprimés ;
f. enrober les comprimés.

14. Procédé de granulation par voie humide pour préparer la composition pharmaceutique de monohydrate d'entécavir selon l'une quelconque des revendications précédentes, comprenant les étapes suivantes :
a. ajouter de l'hydroxypropyl méthylcellulose, de l'amidon et 20 % en poids de mannitol et d'entécavir dans un granulateur et les mélanger pendant 10 min ;
b. ajouter de l'eau à ce mélange et mélanger pour former des granules ;
c. tamiser et sécher les granules humides ;
d. ajouter de la cellulose microcristalline, du glycolate d'amidon sodique et le reste du mannitol et les mélanger pendant 10 min ;
e. ajouter du dioxyde de silicium colloïdal et du fumarate de sodium et de stéaryle à ce mélange et les mélanger jusqu'à obtention d'un mélange de poudres homogène ;
f. comprimer le mélange mélangé pour former des comprimés ;
g. enrober les comprimés.

15. Composition pharmaceutique de monohydrate d'entécavir selon l'une quelconque des revendications précédentes pour une utilisation dans le traitement d'une infection par le virus de l'hépatite B.
